# EUROPEAN PATENT APPLICATION

(11) **EP 4 755 435 A1**
(43) Date of publication of application: **10.06.2026**
(21) Application number: 24868067.0
(22) Date of filing: 29.08.2024
(51) Int. Cl.: A61M 25/09

(54) **GUIDE WIRE**

(30) Priority: 21.09.2023 JP 2023154218
(71) Applicant: Terumo Kabushiki Kaisha, Tokyo 151-0072 (JP)
(72) Inventor: KATSUMOTO, Megumi, Fujinomiya-shi, Shizuoka 418-0015 (JP)
(74) Representative: Casalonga
(86) International application number: PCT/JP2024/030886
(87) International publication number: WO 2025/062987

(57) **Abstract**

To suppress the occurrence of blood vessel damage when advancing through a blood vessel.

Provided is a guide wire 100 including: an elongated wire body 110; and a lubricious coating layer 120 formed of a material capable of being swollen when wetted and covering at least a part of the wire body 110, in which the wire body 110 includes a core member 10, a tubular member 20 disposed so as to cover an outer periphery of a distal portion of the core member 10, and a holding portion 40 holding a part of an inner peripheral surface between a distal end and a proximal end of the tubular member 20, and the lubricious coating layer 120 includes a bulge portion 121 formed on an outer peripheral surface of the wire body 110 at a position of the holding portion 40 by changing a thickness of the lubricious coating layer 120.

## Description

### Technical Field

The present invention relates to a guide wire.

### Background Art

The guide wire is a medical device used to guide various catheters used for treating lesions occurring within blood vessels. The guide wire includes a core shaft (core member), a coil body (tubular member) disposed so as to cover an outer periphery of a distal portion of the core shaft, and joint portions (a distal joint portion, a proximal joint portion, and an intermediate joint portion) that respectively join the distal portion, the proximal portion, and the intermediate portion of the coil body, the intermediate portion being between the distal portion and the proximal portion, to the core shaft (for example, refer to Patent Literature 1).

### Citation List

### Patent Literature

Patent Literature 1: WO 2017/221425 A1

### Summary of Invention

### Technical Problem

When the guide wire of Patent Literature 1 advances through a blood vessel, the guide wire may easily bend at a peripheral portion of the intermediate joint portion as a starting point, and the intermediate fixing portion may come into contact with a blood vessel wall. The intermediate joint portion fixes a coil body, which is a tubular member, to the core member using a fixing material such as solder or a brazing material, and has higher rigidity and higher hardness than the peripheral portion of the intermediate joint portion. Therefore, the intermediate joint portion may come into contact with the blood vessel wall and damage the blood vessel wall when the guide wire is pushed with unnecessarily strong force.

At least one embodiment of the present invention has been made in view of the above circumstances, and an object of the present invention is to provide a guide wire capable of suppressing occurrence of blood vessel damage when advancing in a blood vessel.

### Solution to Problem

The object of the present invention is achieved by any one of the following (1) to (9).
(1) A guide wire including: an elongated wire body; and a lubricious coating layer formed of a material capable of being swollen when wetted and covering at least a part of the wire body, in which the wire body includes a core member, a tubular member disposed so as to cover an outer periphery of a distal portion of the core member, and a holding portion holding a part of an inner peripheral surface between a distal end and a proximal end of the tubular member, and the lubricious coating layer includes a bulge portion formed on an outer peripheral surface of the wire body at a position of the holding portion by changing a thickness of the lubricious coating layer.
(2) The guide wire according to (1), in which the holding portion is an intermediate fixing portion that fixes the tubular member to the core member between a distal fixing portion that fixes the distal end of the tubular member to the core member and a proximal fixing portion that fixes the proximal end of the tubular member to the core member.
(3) The guide wire according to (1), in which the holding portion is a protruding portion that is formed as a part of the core member and protrudes radially outward so as to hold the tubular member by contacting the inner peripheral surface of the tubular member.
(4) The guide wire according to any one of (1) to (3), in which the bulge portion is provided in a region from the distal end to the proximal end of the holding portion in a longitudinal direction.
(5) The guide wire according to any one of (1) to (4), in which the lubricious coating layer has at least one of a distal coating portion covering the distal end of the holding portion or a proximal coating portion covering the proximal end of the holding portion in a lumen of the tubular member.
(6) The guide wire according to any one of (1) to (5), in which the tubular member has a gap portion that allows communication between the outer peripheral surface of the tubular member and a lumen at at least one of a position adjacent to the distal end of the holding portion or a position adjacent to the proximal end of the holding portion.
(7) The guide wire according to any one of (1) to (6), in which the tubular member is a coil formed by winding a wire member so as to cover the outer periphery of the core member.
(8) The guide wire according to (7), in which the coil has a loosely wound portion at at least one of a position adjacent to the distal end of the holding portion and a position adjacent to the proximal end of the holding portion.
(9) The guide wire according to any one of (1) to (8), in which the tubular member includes a distal-side tubular member and a proximal-side tubular member, and the holding portion holds a proximal portion of the distal-side tubular member and a distal portion of the proximal-side tubular member.

### Advantageous Effects of Invention

According to an embodiment of the present invention, in the guide wire, the bulge portion constituted by the lubricious coating layer is disposed on the outer peripheral surface of the holding portion that holds the tubular member from the inside at the intermediate portion between the distal end and the proximal end of the wire body. The bulge portion is formed by changing the thickness of the lubricious coating layer and has the outer peripheral surface including a curved surface that is convex radially outward of the wire body. Therefore, even when the guide wire is bent at the periphery of the holding portion, which has higher rigidity and is harder than the peripheral portion, in the blood vessel and contacts the blood vessel wall, the bulge portion of the lubricious coating layer, which has appropriate elasticity, can mitigate the impact at the time of contact. Therefore, the guide wire can suppress the occurrence of blood vessel damage.

### Brief Description of Drawings

Fig. 1 is a partially enlarged cross-sectional view of a periphery of a distal portion of a guide wire according to the present invention.
Fig. 2 is a schematic cross-sectional view of a guide wire according to the present invention.
Fig. 3 is a partially enlarged view of a periphery of an intermediate fixing portion of a guide wire according to the present invention.
Fig. 4 is a diagram illustrating a shape of a bulge portion of a lubricious coating layer.
Fig. 5A is a view illustrating a form in which a distal coating portion is formed in a holding portion.
Fig. 5B is a view illustrating a form in which a distal coating portion and a proximal coating portion are not formed in a holding portion.
Fig. 6 is a partially enlarged cross-sectional view of a periphery of a distal portion of a guide wire according to a first modification example.
Fig. 7 is a partially enlarged cross-sectional view of a periphery of a distal portion of a guide wire according to a second modification example.
Fig. 8 is a partially enlarged cross-sectional view illustrating another form of a guide wire according to the second modification example.
Fig. 9 is a partially enlarged cross-sectional view of a periphery of a distal portion of a guide wire according to a third modification example.

### Description of Embodiments

Hereinafter, a mode for carrying out the present invention will be described in detail with reference to the drawings. Embodiments herein described are illustrated to embody the technical idea of the present invention and do not limit the present invention. Other embodiments, examples, technical operations, and the like that could be conceived by those skilled in the art without departing from the gist of the present invention are all included in the scope and gist of the invention and included in the invention disclosed in the claims and the scope of equivalents thereof.

Furthermore, for the purpose of illustration and for ease of comprehension, the scale, aspect ratio, shape, and the like in the drawings attached may be changed from actual ones as appropriate and illustrated schematically. However, it is noteworthy that the drawings are examples and do not limit the interpretation of the present invention.

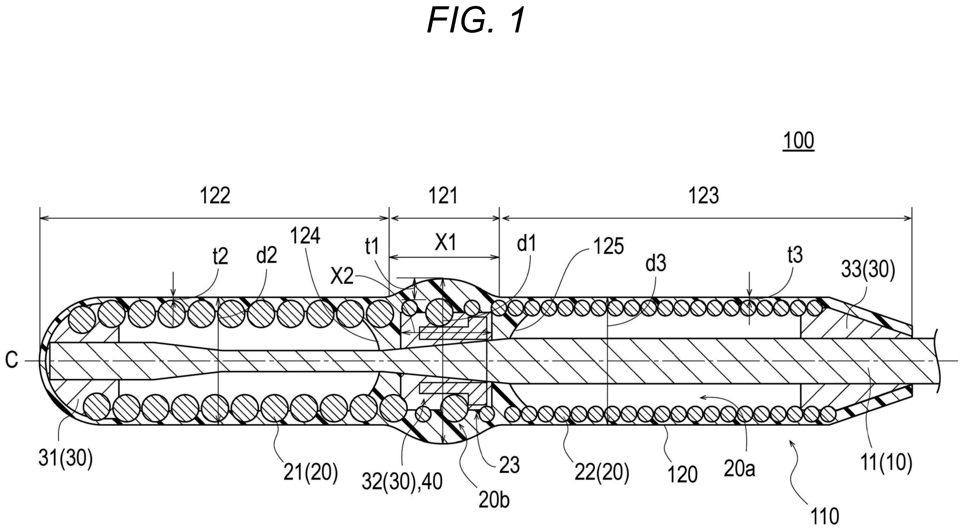

In the present specification, the following directions are defined for convenience of description. In Fig. 1, a "longitudinal direction" is a direction along the central axis C of a guide wire 100. A "radial direction" is a direction away from or approaching the central axis C in an axial orthogonal cross-section (transverse cross-section) with the central axis C of the guide wire 100 as a reference axis. A "circumferential direction" is a rotational direction with the central axis C of the guide wire 100 as a reference axis.

Furthermore, a side on which the guide wire 100 is inserted into a blood vessel is referred to as a "distal side", and a side opposite to the distal side (side gripped by the operator) is referred to as a "proximal side". Furthermore, a portion including a certain range in a longitudinal direction from a distal end (distal-most end) is referred to as a "distal portion", and a portion including a certain range in the longitudinal direction from a proximal end (proximal-most end) is referred to as a "proximal portion".

Note that in the following description, ordinal numbers such as "first" and "second" are used for convenience and do not define any order unless otherwise specified.

The guide wire 100 according to the present invention is a medical device inserted into the blood vessel in order to guide a catheter or a stent for performing intravascular treatment to a lesion. Note that the guide wire 100 can also be used by being inserted into a biological lumen other than a blood vessel (such as a vessel, a ureter, a bile duct, a fallopian tube, a hepatic duct, or the like) in accordance with a therapeutic purpose.

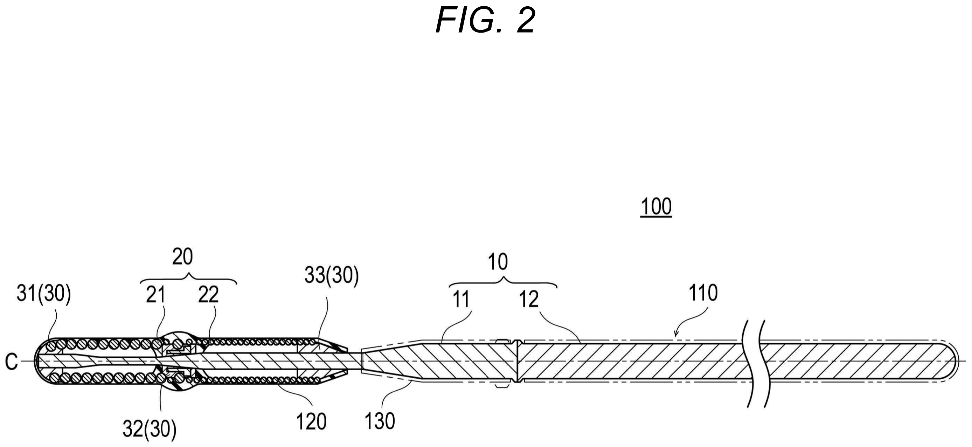

As illustrated in Figs. 1 and 2, the guide wire 100 includes an elongated wire body 110 and a lubricious coating layer 120 covering at least a part of the wire body 110.

### <Wire body>

As illustrated in Fig. 1 or 2, the wire body 110 constitutes a body portion of the guide wire 100, and the lubricious coating layer 120 is formed on at least a part of the outer peripheral surface. The wire body 110 includes a core member 10, a tubular member 20 that is disposed to cover the outer periphery of the distal portion of the core member 10, a fixing portion 30 that fixes a tubular member 20 to the core member 10, and a holding portion 40 that holds a part of the inner peripheral surface between the distal end and the proximal end of the tubular member 20.

The core member 10 includes a first core portion 11 and a second core portion 12 disposed on the proximal side of the first core portion 11 and joined to the first core portion 11. The first core portion 11 is an elongated member extending along the longitudinal direction from the distal end of the second core portion 12 toward the distal side of the guide wire 100. The second core portion 12 is an elongated member extending from the proximal end of the first core portion 11 to the proximal side of the guide wire 100. The first core portion 11 and the second core portion 12 can be joined by welding, brazing, or soldering. The first core portion 11 and the second core portion 12 may be connected using a cylindrical connection member. Note that the core member 10 may be formed of one continuous member.

The core member 10 can be formed of a known material applicable to a guide wire made of a superelastic alloy such as a nickel-titanium-based alloy or stainless steel. The first core portion 11 is preferably formed of a material having lower rigidity than that of the material of the second core portion 12. Note that the first core portion 11 and the second core portion 12 may be formed of the same material.

The core member 10 includes a proximal side coating layer 130 that covers at least a part of the outer peripheral surface. In the present embodiment, the proximal side coating layer 130 is provided on the proximal portion of the first core portion 11 and the outer peripheral surface of the second core portion 12. The proximal side coating layer 130 can be formed of a low-friction material such as a fluorine-based resin (PTFE, ETFE, or the like) or a silicone resin.

As illustrated in Fig. 1, the tubular member 20 has a lumen 20a and is disposed to cover the outer periphery of the core member 10. The tubular member 20 is held from the inside by the holding portion 40 at a position between the distal end and the proximal end of the tubular member 20. Furthermore, as illustrated in Fig. 1, the tubular member 20 has a gap portion 23 that communicates the outer peripheral surface of the tubular member 20 and the lumen 20a at least at a position adjacent to the distal end of the holding portion 40 and a position adjacent to the proximal end of the holding portion 40.

In the present embodiment, the tubular member 20 is a coil formed by spirally winding a wire member around the core member 10. The tubular member 20 is formed of a first coil 21 (distal-side tubular member) disposed at the distal portion of the first core portion 11 and a second coil 22 (proximal-side tubular member) disposed on the proximal side of the first coil 21 and disposed coaxially with the first core portion 11. The first core portion 11 and the second coil 22 surround the first core portion 11 of the core member 10 and are fixed to the first core portion 11. The outer diameters of the first coil 21 and the second coil 22 are preferably constant from the distal end to the proximal end. Note that the tubular member 20 may be formed of one coil or three or more coils.

The outer diameter of the wire member forming the first coil 21 is greater than the outer diameter of the wire member forming the second coil 22. The cross-sectional shape of the wire member forming the first coil 21 and the second coil 22 is preferably circular, but may be elliptical or polygonal. Furthermore, the wire member forming the first coil 21 and the second coil 22 may be not only one wire member but also a stranded wire formed of two or more wire members.

The wire member forming the first coil 21 and the second coil 22 of the tubular member 20 can be formed of a material such as stainless steel, a superelastic alloy, a cobalt-based alloy, a metal such as gold, platinum, or tungsten, or an alloy containing these metals. In particular, the wire member forming the first coil 21 is preferably formed of a radiopaque material such as platinum. Thus, the visibility of the guide wire 100 under fluoroscopy is improved.

The material, the outer diameter, the cross-sectional shape of the wire member forming the first coil 21 and the second coil 22 of the tubular member 20, the pitch of the first coil 21 and the second coil 22, and the like can be appropriately selected in accordance with the purpose of the guide wire 100.

A loosely wound portion 20b, which is a portion having a gap between wire members of the coil, is formed at each of the proximal portion of the first coil 21 and the distal portion of the second coil 22 forming the tubular member 20. The first coil 21 and the second coil 22 are connected by partially entangling their loosely wound portions 20b with each other. Thus, the tubular member 20 has a gap portion 23 that communicates the outer peripheral surface of the tubular member 20 and the lumen 20a. Since the tubular member 20 has the gap portion 23, the material for forming the lubricious coating layer 120 easily enters the lumen 20a of the tubular member 20. Note that the tubular member 20 may have the gap portion 23 between the wire member forming the first coil 21 and the wire member forming the second coil 22 at the connection portion between the first coil 21 and the second coil 22.

The tubular member 20 may be a pipe formed of metal or resin. In a case where the tubular member 20 is a pipe, the gap portion 23 is formed as a hole or a slit penetrating the wall of the pipe.

The fixing portion 30 is a member for fixing the tubular member 20 to the core member 10. The fixing portion 30 includes a distal fixing portion 31 that fixes the distal end of the tubular member 20 to the core member 10, an intermediate fixing portion 32 that fixes the intermediate portion of the tubular member 20 to the core member 10, and a proximal fixing portion 33 that fixes the proximal end of the tubular member 20 to the core member 10.

The intermediate fixing portion 32 is provided at a position where the proximal portion of the first coil 21 and the distal portion of the second coil 22 are entangled with each other on the first core portion 11. The intermediate fixing portion 32 fixes the proximal portion of the first coil 21 and the distal portion of the second coil 22 to the first core portion 11 via a cylindrical member formed of metal, a resin material, or the like. The cylindrical member is disposed between the inner peripheral surface of the tubular member 20 and the outer peripheral surface of the core member 10, and a gap between the inner peripheral surface of the tubular member 20 and the outer peripheral surface of the core member 10 is reduced to easily fix the tubular member 20 and the core member 10 coaxially. The intermediate fixing portion 32 holds the tubular member 20 from the inside. The intermediate fixing portion 32 is provided at a position approximately 30 mm from the distal end of the guide wire 100.

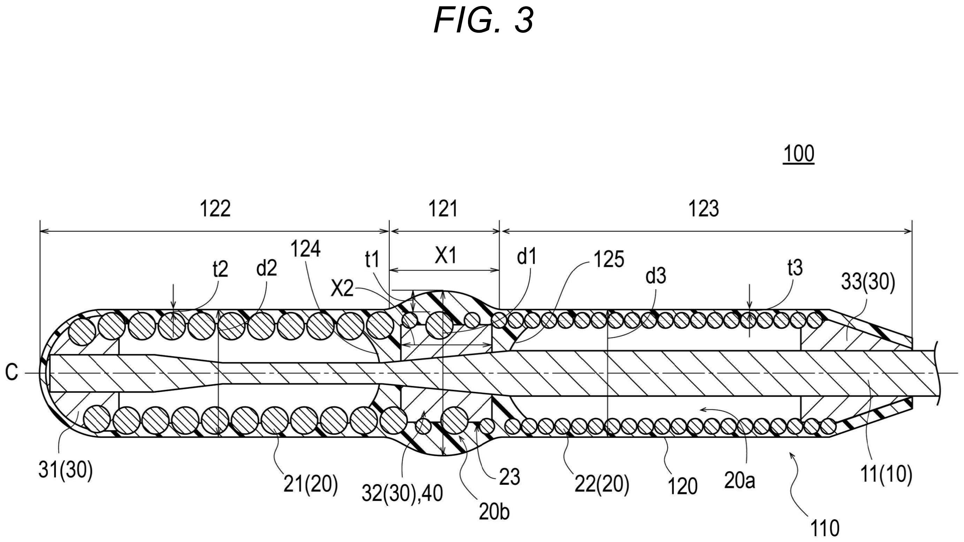

Note that as illustrated in Fig. 3, the intermediate fixing portion 32 may be formed of only a fixing material such as solder or brazing material. The intermediate fixing portion 32 is disposed between the outer peripheral surface of the core member 10 and the inner peripheral surface of the tubular member 20, and fixes both. Thus, the tubular member 20 is held by the intermediate fixing portion 32.

The fixing material forming the fixing portion 30 is a brazing material or solder. Examples of the brazing material include a gold brazing material and a silver brazing material. Examples of the solder include tin-silver alloy solder and tin-lead alloy solder. The fixing material may be an adhesive.

As illustrated in Fig. 1, the holding portion 40 is provided at a position between the distal end and the proximal end of the tubular member 20, and at least partially comes into contact with the inner peripheral surface of the tubular member 20 to hold the tubular member 20 from the inside. In the guide wire 100 according to the present embodiment, the intermediate fixing portion 32 corresponds to the holding portion 40. A plurality of the holding portions 40 may be formed along the longitudinal direction between the distal end and the proximal end of the tubular member 20.

In the guide wire 100, as illustrated in Fig. 1, the gap portion 23 of the tubular member 20 is formed at a position adjacent to the distal end of the holding portion 40 and at a position adjacent to the proximal end of the holding portion 40. Note that the gap portion 23 may be formed at a position adjacent to either the distal end or the proximal end of the holding portion 40.

The lubricious coating layer 120 covers each of the portions (the tubular member 20 and the fixing portion 30) provided on the first core portion 11 and a part of the first core portion 11.

At least a part of the lubricious coating layer 120 is formed of a material that can be swollen when wetted. Such a lubricious coating layer 120 can be formed of a hydrophilic polymer. Examples of the hydrophilic polymer forming the lubricious coating layer 120 include a cellulose-based polymer substance, a polyethylene oxide-based polymer substance, a maleic anhydride-based polymer substance (for example, a maleic anhydride copolymer such as a methyl vinyl ether-maleic anhydride copolymer), an acrylamide-based polymer substance (for example, a block copolymer of polyacrylamide and glycidyl methacrylate-dimethylacrylamide), water-soluble nylon, polyvinyl alcohol, polyvinyl pyrrolidone, and derivatives thereof. In particular, the lubricious coating layer 120 is preferably formed of a material that is swollen to form a hydrogel when wetted. Since the hydrogel has moderate elasticity, it is possible to alleviate the impact when the guide wire 100 and the blood vessel wall come into contact with each other.

The lubricious coating layer 120 includes a bulge portion 121, a first coating portion 122, a second coating portion 123, a distal coating portion 124, and a proximal coating portion 125.

### <Bulge portion>

As illustrated in Fig. 1, the bulge portion 121 is a portion where the thickness of the lubricious coating layer 120 is greater. The bulge portion 121 is formed by changing the thickness of the lubricious coating layer 120 and has an outer peripheral surface including a curved surface that is convex radially outward of the guide wire 100. The bulge portion 121 is provided at the position of the holding portion 40 of the guide wire 100 in the longitudinal direction. In a case where a plurality of the holding portions 40 are formed, the bulge portions 121 may be disposed corresponding to the positions of the holding portions 40.

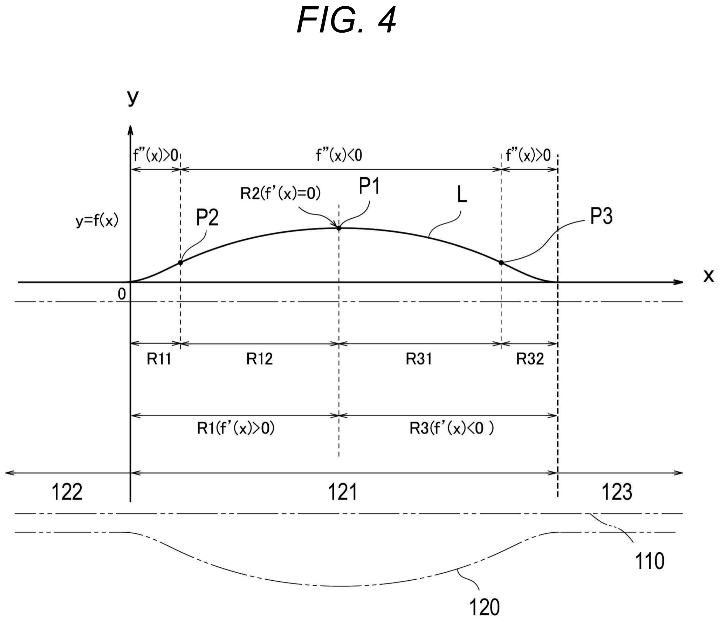

As illustrated in Fig. 4, the bulge portion 121 includes a first region R1 in which the thickness of the bulge portion 121 increases from the distal end toward the proximal end of the guide wire 100, a second region R2 which is continuous with the proximal end of the first region R1 and in which the outer diameter of the bulge portion 121 is the maximum, and a third region R3 which is continuous with the proximal end of the second region R2 and in which the thickness of the bulge portion 121 decreases from the distal end toward the proximal end. Note that in the present specification, the thickness of the lubricious coating layer 120 is a thickness in a state in which the material for forming the lubricious coating layer 120 is swollen when wetted.

Fig. 4 is a diagram in which the central axis C of the guide wire 100 placed in a natural state is superimposed on a two-axis orthogonal coordinate system in which an axis along the central axis C of the guide wire 100 is an x axis (longitudinal direction of the guide wire 100) and an axis orthogonal to the x axis and in contact with the distal-most end of the bulge portion 121 is a y axis (radial direction of the guide wire 100) in a state in which the central axis C of the guide wire 100 is parallel to the x axis and the distal-most end of the first region R1 of the guide wire 100 is aligned with the origin. The bulge portion 121 has a three-dimensional shape obtained by rotating the curve L formed by the outer peripheral surface of the bulge portion 121 represented in the first quadrant of the orthogonal coordinate system illustrated in Fig. 4 about the central axis C as an axis of rotation.

As illustrated in Fig. 4, in the first quadrant of the orthogonal coordinate system, in a case where a function representing the curve L formed by the outer peripheral surface of the bulge portion 121 when the guide wire 100 is viewed from the side is y=f(x), the function y=f(x) has a second derivative f"(x) in a certain interval of x (x>0). In Fig. 4, the first region R1 of the bulge portion 121 corresponds to an interval of x satisfying f' (x)>0 on the distal side of the local maximum point P1 (first derivative f' (x)=0) on the curve L where the function y=f(x) is the global and local maximum. The second region R2 of the bulge portion 121 corresponds to an interval of x satisfying f' (x)=0 (local maximum point P1) in Fig. 4. The third region R3 of the bulge portion 121 corresponds to an interval of x satisfying f' (x)<0 on the proximal side of the local maximum point P1 on the curve L in Fig. 4.

The first region R1 of the bulge portion 121 includes a first region distal portion R11 that is convex radially inward in a predetermined range extending from the distal end of the first region R1 on the distal side of the first region R1, and a first region proximal portion R12 that is convex radially outward on the proximal side of the first region distal portion R11. The first region R1 of the bulge portion 121 has a first inflection point P2 where f"(x)=0 and the sign of f"(x) is inverted and the unevenness of the curve L changes on the curve L in Fig. 4. The first region distal portion R11 corresponds to an interval where the function y=f(x) is convex downward and satisfies f' (x)>0 and f"(x)>0 with the first inflection point P2 on the curve L in Fig. 4 as a boundary, and the first region proximal portion R12 corresponds to an interval where the function y=f(x) is convex upward and satisfies f' (x)>0 and f"(x)<0. Note that in Fig. 4, "convex upward" has a characteristic that a line segment connecting any two points on the curve L is always below the curve. On the other hand, "convex downward" has a characteristic that a line segment connecting any two points on the curve L is always above the curve.

The second region R2 of the bulge portion 121 is a portion where the bulge portion 121 has the maximum outer diameter. The second region R2 is connected to the first region proximal portion R12 of the first region R1 and the third region distal portion R31 of the third region R3. In Fig. 4, the second region R2 exists as a boundary point between the first region R1 and the third region R3, and coincides with the local maximum point P1 on the curve L where the function y=f(x) is the global and local maximum. The second region R2 satisfies f' (x)=0 in Fig. 4.

The third region R3 of the bulge portion 121 includes a third region distal portion R31 that is convex radially outward in a predetermined range extending from the proximal end of the second region R2 on the distal side of the third region R3, and a third region proximal portion R32 that is convex radially inward on the proximal side of the third region distal portion R31. The third region R3 of the bulge portion 121 has a second inflection point P3 where f"(x)=0 and the sign of f"(x) is inverted and the unevenness of the curve L changes on the curve L in Fig. 4. The third region distal portion R31 corresponds to an interval where the function y=f(x) is convex upward and satisfies f' (x)<0 and f"(x)<0 with the second inflection point P3 on the curve L in Fig. 4 as a boundary, and the third region proximal portion R32 corresponds to an interval where the function y=f(x) is convex downward and satisfies f' (x)<0 and f"(x)>0.

In the lubricious coating layer 120, the outer peripheral surface of the bulge portion 121 and the outer peripheral surface of the first coating portion 122 are smoothly connected by the first region distal portion R11 located at the connection portion between the bulge portion 121 and the first coating portion 122. In the lubricious coating layer 120, the outer peripheral surface of the bulge portion 121 and the outer peripheral surface of the second coating portion 123 are smoothly connected by the third region proximal portion R32 located at the connection portion between the bulge portion 121 and the second coating portion 123. Thus, in the guide wire 100, the connection portion between the bulge portion 121 and the first coating portion 122 and the connection portion between the bulge portion 121 and the second coating portion 123 of the lubricious coating layer 120 are less likely to be caught on the blood vessel wall or the lumen of the catheter, and thus, it is possible to suppress peeling of the lubricious coating layer 120.

The bulge portion 121 has an outer peripheral surface including a curved surface that is convex radially outward and which is formed by changing the thickness of the material that can be swollen when wetted, and covers the outer peripheral surface of the wire body 110 at least at the position of the holding portion 40 of the guide wire 100. In other words, the bulge portion 121 covers the outer peripheral surface of the holding portion 40 of the wire body 110 and/or the outer peripheral surface of the tubular member 20 at the position of the holding portion 40. Thus, the bulge portion 121 can reduce the impact caused when the guide wire 100 and the blood vessel wall come into contact with each other and suppress the occurrence of blood vessel damage.

The bulge portion 121 is preferably provided so as to cover the entire holding portion 40. That is, the length X1 along the longitudinal direction of the bulge portion 121 is longer than the length X2 along the longitudinal direction of the holding portion 40, and is preferably provided so as to cover at least a region from the distal end to the proximal end of the holding portion 40. Note that at least a part of the bulge portion 121 is only required to be formed on the outer peripheral surface of the wire body 110 at the position of the holding portion 40. The length X1 along the longitudinal direction of the bulge portion 121 is formed to be preferably 15% or more and 125% or less, more preferably 75% or more and 125% or less, and still more preferably 100% or more and 125% or less with respect to the length X2 along the longitudinal direction of the holding portion 40.

### <First coating portion>

As illustrated in Fig. 1, the first coating portion 122 is a portion located on the distal side of the bulge portion 121 of the lubricious coating layer 120. The first coating portion 122 is preferably provided so as to cover the distal end of the wire body 110. That is, the first coating portion 122 is preferably provided so as to cover at least the first coil 21. The first coating portion 122 is formed to have a substantially constant thickness. The outer peripheral surface of the first coating portion 122 is smoothly connected to the outer peripheral surface of the bulge portion 121. Note that the first coating portion 122 may not be provided.

### <Second coating portion>

As illustrated in Fig. 1, the second coating portion 123 is a portion on the proximal side of the bulge portion 121 of the lubricious coating layer 120. The second coating portion 123 is preferably provided so as to cover at least the second coil 22. The second coating portion 123 is formed to have a substantially constant thickness. The outer peripheral surface of the second coating portion 123 is smoothly connected to the outer peripheral surface of the bulge portion 121.

As illustrated in Fig. 1, in a state in which the lubricious coating layer 120 is swollen, the maximum thickness t1 of the bulge portion 121 in the radial direction is greater than the maximum thickness t2 of the first coating portion 122 in the radial direction and the maximum thickness t3 of the second coating portion 123 in the radial direction (t1>t2, t3). Furthermore, in the guide wire 100, the maximum outer diameter d1 of the bulge portion 121 is greater than the maximum outer diameter d2 of the first coating portion 122 and the maximum outer diameter d3 of the second coating portion 123 (d1>d2, d3). Thus, the bulge portion 121 can more effectively exert a buffering effect on the blood vessel wall. Note that the maximum thickness t1 of the bulge portion 121 in the radial direction, the maximum thickness t2 of the first coating portion 122 in the radial direction, and the maximum thickness t3 of the second coating portion 123 in the radial direction are respectively the thicknesses of the lubricious coating layer 120 on the outer peripheral surfaces of the first coil 21 and the second coil 22 (at the outermost positions of the wire members forming the first coil 21 and the second coil 22 in the radial direction).

The maximum thickness t1 of the bulge portion 121 in the radial direction in a state in which the lubricious coating layer 120 is swollen is preferably 3 µm or more and 12 µm or less, and more preferably 5 µm or more and 8 µm or less. Note that the maximum thickness t1 of the bulge portion 121 in the radial direction in a state in which the lubricious coating layer 120 is dried is preferably 0.4 µm or more and 1.2 µm or less, and more preferably 0.8 µm or more and 1.2 µm or less. The maximum thickness t2 of the first coating portion 122 in the radial direction and the maximum thickness t3 of the second coating portion 123 in the radial direction in a state in which the lubricious coating layer 120 is swollen are preferably 1 µm or more and 8 µm or less, and more preferably 2 µm or more and 7 µm or less. Note that the maximum thickness t2 of the first coating portion 122 in the radial direction and the maximum thickness t3 of the second coating portion 123 in the radial direction in a state in which the lubricious coating layer 120 is dried are preferably 0.2 µm or more and 0.8 µm or less, and more preferably 0.5 µm. The maximum thickness t2 of the first coating portion 122 in the radial direction and the maximum thickness t3 of the second coating portion 123 in the radial direction may be substantially equal to each other, but may be different from each other. For example, in a state in which the lubricious coating layer 120 is swollen, it is preferable that the maximum thickness t2 of the first coating portion 122 in the radial direction is smaller than the maximum thickness t3 of the second coating portion 123 in the radial direction, the maximum thickness t2 of the first coating portion 122 in the radial direction is 2 µm or more and 4 µm or less, and the maximum thickness t3 of the second coating portion 123 in the radial direction is 4 µm or more and 7 µm or less.

The lubricious coating layer 120 may be composed of only the bulge portion 121. Furthermore, the lubricious coating layer 120 may have a coating portion other than the bulge portion 121, the first coating portion 122, and the second coating portion 123.

The first coating portion 122 and the second coating portion 123 of the lubricious coating layer 120 are preferably formed of the same material as the bulge portion 121. Note that the bulge portion 121, the first coating portion 122, and the second coating portion 123 may be formed of different materials. For example, the bulge portion 121 and the second coating portion 123 may be formed of a material that can be swollen when wetted, and the first coating portion 122 may be partially formed of silicone.

### <Distal coating portion>

As illustrated in Fig. 1, the distal coating portion 124 is a portion that covers the distal end of the holding portion 40 in the lumen 20a of the tubular member 20. The distal coating portion 124 is connected to the bulge portion 121 via the gap portion 23 of the tubular member 20. Thus, since the lubricious coating layer 120 is firmly fixed to the holding portion 40 and the tubular member 20, peeling of the lubricious coating layer 120 in the vicinity of the distal end of the holding portion 40 is suppressed.

### <Proximal coating portion>

As illustrated in Fig. 1, the proximal coating portion 125 is a portion that covers the proximal end of the holding portion 40 in the lumen 20a of the tubular member 20. The proximal coating portion 125 is connected to the bulge portion 121 via the gap portion 23 of the tubular member 20. Thus, since the lubricious coating layer 120 is firmly fixed to the holding portion 40 and the tubular member 20, peeling of the lubricious coating layer 120 in the vicinity of the proximal end of the holding portion 40 is suppressed.

Since there is a difference in rigidity between the holding portion 40 of the guide wire 100 and the peripheral portion of the holding portion 40, the holding portion 40 is easily bent starting from its peripheral portion, and the bulge portion 121 may be peeled off due to the bending of the holding portion 40. The guide wire 100 can effectively suppress peeling of the bulge portion 121 by providing the distal coating portion 124 and the proximal coating portion 125 on the lubricious coating layer 120 to firmly fix the lubricious coating layer 120 to the holding portion 40 and the tubular member 20.

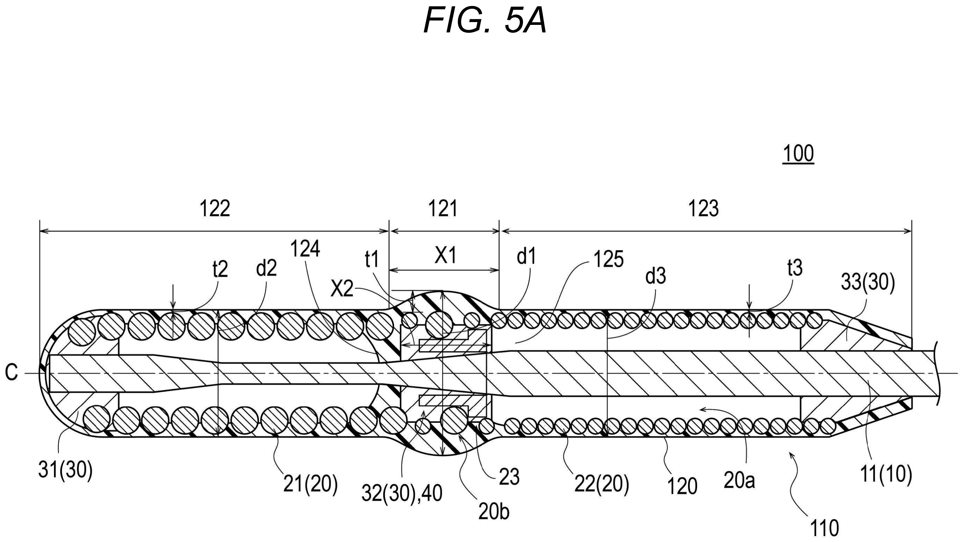

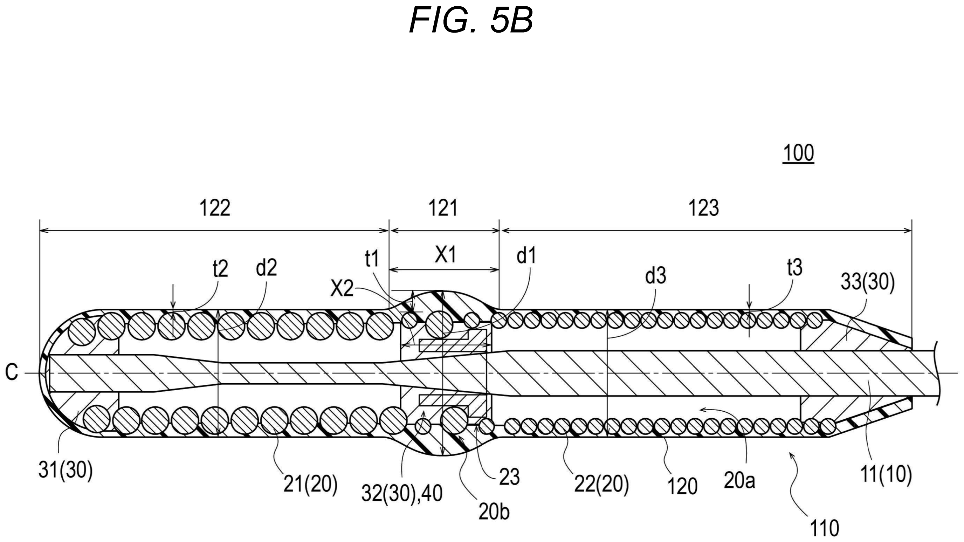

Note that as illustrated in Fig. 5A, the lubricious coating layer 120 may include only at least one of the distal coating portion 124 or the proximal coating portion 125. Alternatively, as illustrated in Fig. 5B, the lubricious coating layer 120 may not include the distal coating portion 124 and the proximal coating portion 125.

The lubricious coating layer 120 can be formed by immersing the wire body 110 in a solution containing a material for forming the lubricious coating layer 120, pulling up the wire body 110 from the solution in the vertical direction, and then drying the wire body. The number of times the wire body 110 is immersed in the solution may be one time or a plurality of times.

When the wire body 110 is pulled up from the solution, a part of the solution adhering to the outer peripheral surface of the wire body 110 forms a liquid pool covering the entire periphery of the distal portion of the wire body 110 due to the action of surface tension. The bulge portion 121 can be formed by drying the liquid pool portion formed at the distal portion of the wire body 110. When the wire body 110 is pulled up from the solution, the solution adhering to the outer peripheral surface of the wire body 110 flows downward due to gravity, such that the first coating portion 122 and the second coating portion 123 can be formed as layers having a substantially constant thickness. The distal coating portion 124 and the proximal coating portion 125 can be formed by allowing a material for forming the lubricious coating layer 120 to flow into the lumen 20a of the tubular member 20 through the gap portion 23 of the tubular member 20. Furthermore, the bulge portion 121 may be formed by applying the material for forming the lubricious coating layer 120 to the outer peripheral surface of the wire body 110 at a position where the bulge portion 121 is intended to be formed.

The first coating portion 122 may be formed by removing the material for forming the bulge portion 121 and the second coating portion 123 from the distal portion of the wire body 110 and applying another material for forming the lubricious coating layer 120 such as silicone to the wire body 110.

### [Modification example]

The guide wire 100 of the present invention can be implemented by being appropriately modified as in the following modification examples. Note that, in the following description of each of the modification examples, differences from the above-described form will be mainly described, and constituent elements having functions equivalent to those of other forms will be denoted by the same or related reference numerals and will not be described in detail. Furthermore, the configuration, the members, the method of use, and the like may be the same as those in each form. Moreover, each modification example can be implemented by appropriately selecting a necessary configuration among the configurations indicated in each modification example and combining the configuration with other forms within a range not departing from the gist of the present invention.

### <First modification example>

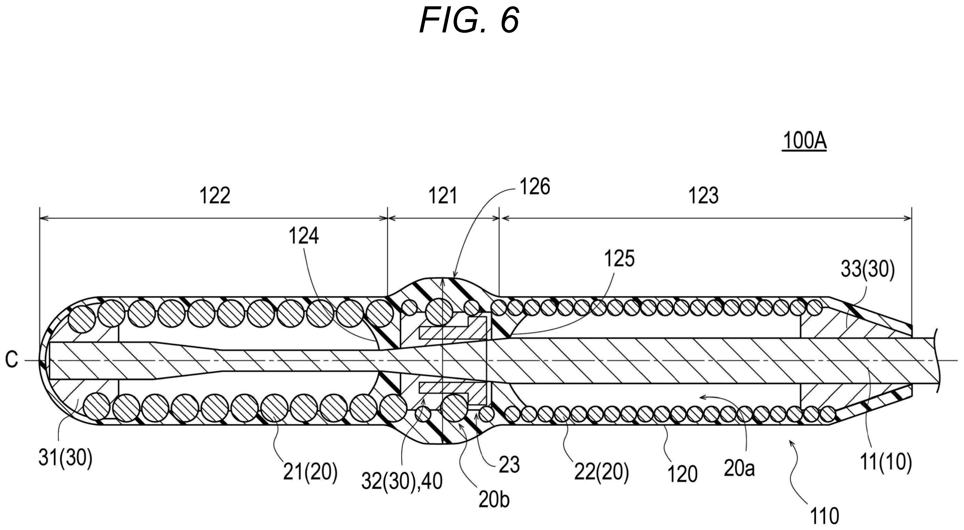

A guide wire 100A according to a first modification example will be described. Fig. 6 is an enlarged cross-sectional view of the periphery of the distal portion of the guide wire 100A according to the first modification example.

As illustrated in Fig. 6, the guide wire 100A includes the lubricious coating layer 120 that covers at least a part of the wire body 110. The guide wire 100A is different from the above-described form in that the second region R2 of the bulge portion 121 of the lubricious coating layer 120 has a flat portion 126 extending for a predetermined length in the longitudinal direction.

In the guide wire 100A, the second region R2 of the bulge portion 121 has the flat portion 126 extending for a predetermined length in the longitudinal direction. The thickness of the bulge portion 121 in the flat portion 126 is substantially constant along the longitudinal direction, and is the maximum thickness t1 of the bulge portion 121 in the radial direction.

By providing the flat portion 126 in the second region R2 of the bulge portion 121, the guide wire 100A can increase the length along the longitudinal direction without excessively enlarging the outer diameter of the bulge portion 121. Therefore, in the guide wire 100A, even in a case where the length of the holding portion 40 along the longitudinal direction is long, the outer diameter of the bulge portion 121 can be set to a size that does not interfere with use.

In the guide wire 100A according to the first modification example, by providing the flat portion 126 in the second region R2 of the bulge portion 121, it is possible to form the bulge portion 121 that covers the entire length of the holding portion 40 while maintaining the outer diameter to the extent that it does not interfere with use.

### <Second modification example>

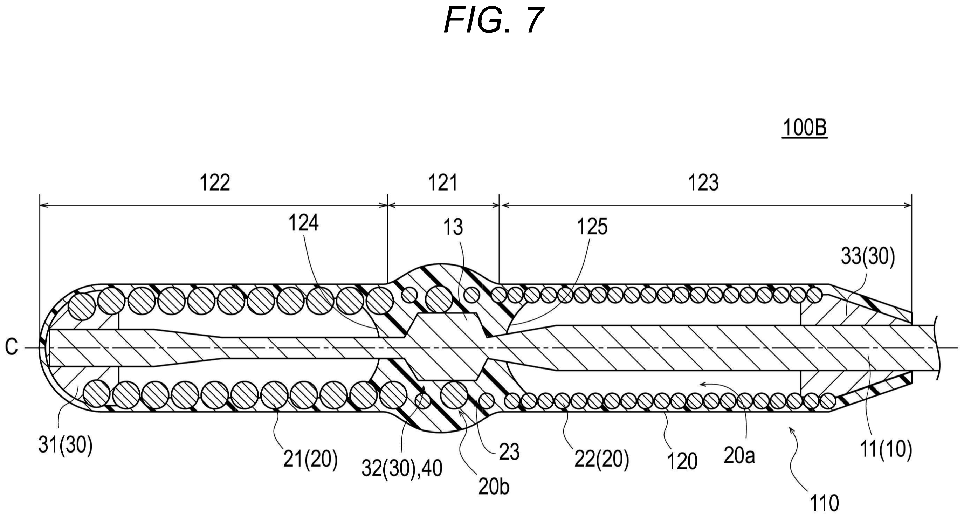

A guide wire 100B according to a second modification example will be described. Fig. 7 is an enlarged cross-sectional view of the periphery of the distal portion of the guide wire 100B according to the second modification example.

As illustrated in Fig. 7, the guide wire 100B includes the lubricious coating layer 120 that covers at least a part of the wire body 110. The guide wire 100B is different from the above-described form in that the holding portion 40 is formed as a part of the core member 10.

As illustrated in Fig. 7, the guide wire 100B includes a protruding portion 13 formed to concentrically protrude radially outward on the first core portion 11. In the guide wire 100B, the protruding portion 13 corresponds to the holding portion 40.

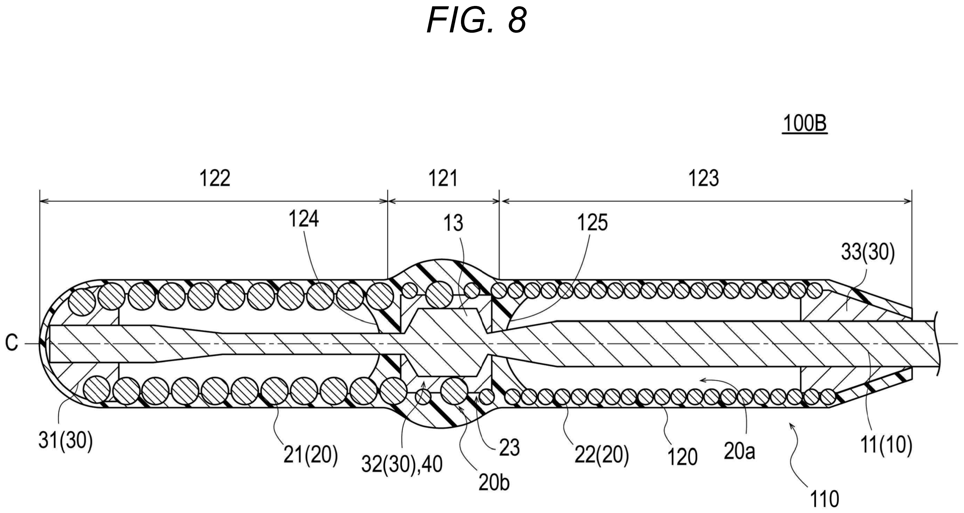

The protruding portion 13 holds the tubular member 20 by contacting the inner peripheral surface of the tubular member 20. The protruding portion 13 is preferably provided at a position corresponding to a connection portion between the first coil 21 and the second coil 22 on the first core portion 11. Note that as illustrated in Fig. 8, the protruding portion 13 may be fixed to the tubular member 20 via the fixing material.

### <Third modification example>

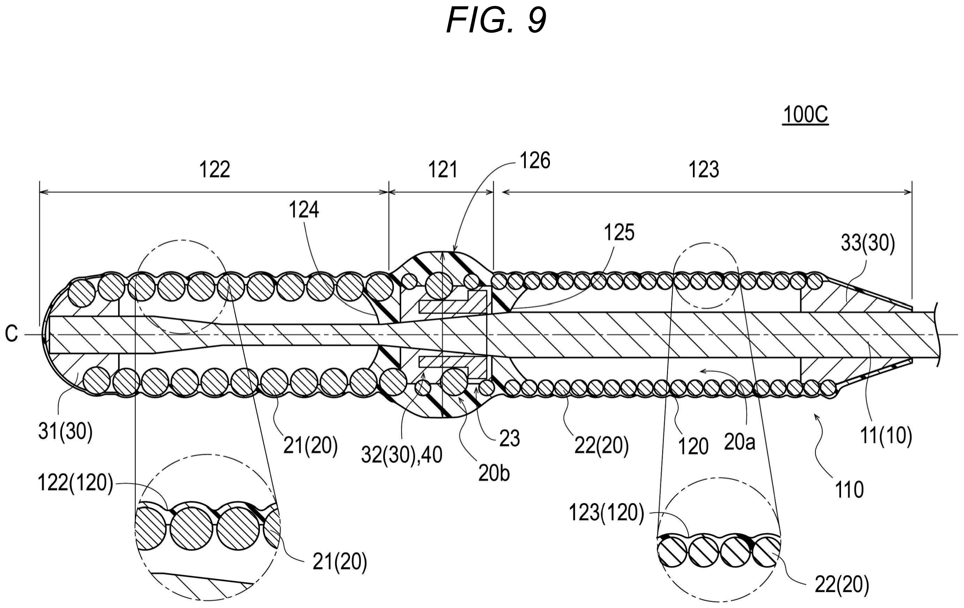

A guide wire 100C according to a third modification example will be described. Fig. 9 is an enlarged cross-sectional view of the periphery of the distal portion of the guide wire 100C according to the third modification example.

As illustrated in Fig. 9, the guide wire 100C is different from the above-described form in the shape of the outer peripheral surface of the lubricious coating layer 120 covering at least a part of the wire body 110.

As illustrated in Fig. 9, in the guide wire 100C, the shape of the outer peripheral surface of the first coating portion 122 can be an uneven shape following the outer periphery of the wire member forming the first coil 21. Furthermore, as illustrated in Fig. 9, in the guide wire 100C, the shape of the outer peripheral surface of the second coating portion 123 can be an uneven shape following the outer periphery of the wire member forming the second coil 22. In the guide wire 100C, in a case where the thickness of the lubricious coating layer 120 is very thin, the outer peripheral surface of the lubricious coating layer 120 covering the tubular member 20 may have an uneven shape following the outer peripheral shape of the tubular member 20(the first coil 21 and the second coil 22) as illustrated in Fig. 9.

As illustrated in Fig. 9, in the guide wire 100C, both the first coating portion 122 and the second coating portion 123 have the above-described uneven shape, but only one of the coating portions may have the uneven shape following the coil shape. That is, in the guide wire 100C, the shape of the outer peripheral surface of the first coating portion 122 may be an uneven shape following the outer peripheral shape of the first coil 21, and the shape of the outer peripheral surface of the second coating portion 123 may be a flat shape along the longitudinal direction. Furthermore, in the guide wire 100C, the shape of the outer peripheral surface of the first coating portion 122 may be a flat shape along the longitudinal direction, and the shape of the outer peripheral surface of the second coating portion 123 may be an uneven shape following the outer peripheral shape of the second coil 22.

### [Operational effects]

As described above, the guide wire 100 according to the present invention includes the elongated wire body 110 and the lubricious coating layer 120 that is formed of a material that can be swollen when wetted and covers at least a part of the wire body 110, the wire body 110 includes the core member 10, the tubular member 20 that is disposed to cover the outer periphery of the distal portion of the core member 10, and the holding portion 40 that holds a part of the inner peripheral surface between the distal end and the proximal end of the tubular member 20, and the lubricious coating layer 120 includes the bulge portion 121 that is formed on the outer peripheral surface of the wire body 110 at the position of the holding portion 40 by changing the thickness of the lubricious coating layer 120.

With such a configuration, the guide wire 100 includes the bulge portion 121 formed of the lubricious coating layer 120 on the outer peripheral surface of the wire body 110 at the position of the holding portion 40 that holds the tubular member 20 from the inside between the distal end and the proximal end of the wire body 110. The bulge portion 121 is formed by changing the thickness of the lubricious coating layer 120 and has the outer peripheral surface including a curved surface that is convex radially outward of the wire body 110. Therefore, even when the guide wire 100 is bent at the periphery of the holding portion 40, which has higher rigidity and is harder than the peripheral portion, in the blood vessel and contacts the blood vessel wall, the bulge portion 121 of the lubricious coating layer 120, which has appropriate elasticity, can mitigate the impact at the time of contact. Therefore, the guide wire 100 can suppress the occurrence of blood vessel damage.

Furthermore, the holding portion 40 of the guide wire 100 may be the intermediate fixing portion 32 that fixes the tubular member 20 to the core member 10 between the distal fixing portion 31 that fixes the distal end of the tubular member 20 to the core member 10 and the proximal fixing portion 33 that fixes the proximal end of the tubular member 20 to the core member 10.

With such a configuration, the guide wire 100 includes the bulge portion 121 formed of the lubricious coating layer 120 on the outer peripheral surface at the position of the intermediate fixing portion 32 that functions as the holding portion 40 holding the tubular member 20 from the inside between the distal end and the proximal end of the wire body 110. The bulge portion 121 is formed by changing the thickness of the lubricious coating layer 120 and has the outer peripheral surface including a curved surface that is convex radially outward of the wire body 110. Therefore, even when the guide wire 100 is bent at the periphery of the intermediate fixing portion 32, which has higher rigidity and is harder than the peripheral portion, in the blood vessel and contacts the blood vessel wall, the bulge portion 121 of the lubricious coating layer 120, which has appropriate elasticity, can mitigate the impact at the time of contact. Therefore, the guide wire 100 can suppress the occurrence of blood vessel damage.

Furthermore, the holding portion 40 of the guide wire 100 may be the protruding portion 13 that is formed as a part of the core member 10 and protrudes radially outward so as to hold the tubular member 20 by contacting the inner peripheral surface of the tubular member 20.

With such a configuration, the guide wire 100 includes the bulge portion 121 formed of the lubricious coating layer 120 on the outer peripheral surface at the position of the protruding portion 13 that functions as the holding portion 40 holding the tubular member 20 from the inside between the distal end and the proximal end of the wire body 110. The bulge portion 121 is formed by changing the thickness of the lubricious coating layer 120 and has the outer peripheral surface including a curved surface that is convex radially outward of the wire body 110. Therefore, even when the guide wire 100 is bent at the periphery of the protruding portion 13, which has higher rigidity and is harder than the peripheral portion, in the blood vessel and contacts the blood vessel wall, the bulge portion 121 of the lubricious coating layer 120, which has appropriate elasticity, can mitigate the impact at the time of contact. Therefore, the guide wire 100 can suppress the occurrence of blood vessel damage.

Furthermore, the bulge portion 121 of the guide wire 100 may be provided in a region from the distal end to the proximal end of the holding portion 40 in the longitudinal direction.

With such a configuration, in the guide wire 100, since the holding portion 40 is covered with the bulge portion 121 of the lubricious coating layer 120 over the entire length, it is possible to effectively suppress the occurrence of blood vessel damage.

Furthermore, the lubricious coating layer 120 of the guide wire 100 may be configured to have at least one of the distal coating portion 124 covering the distal end of the holding portion 40 or the proximal coating portion 125 covering the proximal end of the holding portion 40 in the lumen 20a of the tubular member 20.

With such a configuration, in the guide wire 100, the lubricious coating layer 120 can be firmly fixed to the tubular member 20, the holding portion 40, the core member 10, and the like on the distal side and/or the proximal side of the holding portion 40, and thus, it is possible to suppress peeling of the bulge portion 121 from the wire body 110.

Furthermore, the tubular member 20 of the guide wire 100 may be configured to have the gap portion 23 that allows communication between the outer peripheral surface of the tubular member 20 and the lumen at at least one of a position adjacent to the distal end of the holding portion 40 or a position adjacent to the proximal end of the holding portion 40.

With such a configuration, in the guide wire 100, the material for forming the lubricious coating layer 120 easily enters the lumen 20a of the tubular member 20 from the gap portion 23. Therefore, in the guide wire 100, the distal coating portion 124 covering the distal end of the holding portion 40 and the proximal coating portion 125 covering the proximal end of the holding portion 40 can be easily formed.

Furthermore, the tubular member 20 of the guide wire 100 may be a coil wound so as to cover the outer periphery of the core member 10.

With such a configuration, the flexibility of the distal portion of the guide wire 100 is improved.

Furthermore, the coil may be configured to have the loosely wound portion 20b at at least one of the position adjacent to the distal end of the holding portion 40 or the position adjacent to the proximal end of the holding portion 40.

With such a configuration, the guide wire 100 has the loosely wound portion 20b in at least one of the position adjacent to the distal end of the holding portion 40 of the coil, which is the tubular member 20, or the position adjacent to the proximal end of the holding portion 40, and thus, the gap portion 23 can be easily formed only by changing the pitch of the coil.

Furthermore, the tubular member 20 of the guide wire 100 includes a distal-side tubular member (the first coil 21) and a proximal-side tubular member (the second coil 22), and the holding portion 40 holds the proximal portion of the distal-side tubular member and the distal portion of the proximal-side tubular member.

With such a configuration, since the guide wire 100 includes the distal-side tubular member (the first coil 21) and the proximal-side tubular member (the second coil 22), the physical properties can be changed at the distal side and the proximal side of the tubular member 20. Therefore, the guide wire 100 can improve flexibility of the distal portion and visibility under fluoroscopy. Furthermore, in the guide wire 100, since the bulge portion 121 is provided in the holding portion 40 that is the connection portion between the distal-side tubular member and the proximal-side tubular member, it is possible to suppress the occurrence of blood vessel damage.

The present application is based on Japanese Patent Application No. 2023-154218 filed on September 21, 2023, the disclosure content of which is incorporated herein by reference in its entirety.

### Reference Signs List

- 10: Core member
- 11: First core portion
- 12: Second core portion
- 13: Protruding portion
- 20: Tubular member (20a lumen, 20b loosely wound portion)
- 21: First coil
- 22: Second coil
- 23: Gap portion
- 30: Fixing portion
- 31: Distal fixing portion
- 32: Intermediate fixing portion
- 33: Proximal fixing portion
- 40: Holding portion
- 100, 100A, 100B, 100C: Guide wire
- 110: Wire body
- 120: Lubricious coating layer
- 121: Bulge portion
- 122: First coating portion
- 123: Second coating portion
- 124: Distal coating portion
- 125: Proximal coating portion
- 126: Flat portion
- 130: Proximal side coating layer
- C: Center axis
- d1: Maximum outer diameter of bulge portion
- d2: Maximum outer diameter of first coating portion
- d3: Maximum outer diameter of second coating portion
- R1: First region
- R2: Second region
- R3: Third region
- R11: First region distal portion
- R12: First region proximal portion
- R31: Third region distal portion
- R32: Third region proximal portion
- t1: Maximum thickness of bulge portion in radial direction
- t2: Maximum thickness of first coating portion in radial direction
- t3: Maximum thickness of second coating portion in radial direction
- X1: Length of bulge portion in longitudinal direction
- X2: Length of holding portion in longitudinal direction

## Claims

1. A guide wire comprising: an elongated wire body; and a lubricious coating layer formed of a material capable of being swollen when wetted and covering at least a part of the wire body,
wherein the wire body includes a core member, a tubular member disposed so as to cover an outer periphery of a distal portion of the core member, and a holding portion holding a part of an inner peripheral surface between a distal end and a proximal end of the tubular member, and
the lubricious coating layer includes a bulge portion formed on an outer peripheral surface of the wire body at a position of the holding portion by changing a thickness of the lubricious coating layer.

2. The guide wire according to claim 1, wherein the holding portion is an intermediate fixing portion that fixes the tubular member to the core member between a distal fixing portion that fixes the distal end of the tubular member to the core member and a proximal fixing portion that fixes the proximal end of the tubular member to the core member.

3. The guide wire according to claim 1, wherein the holding portion is a protruding portion that is formed as a part of the core member and protrudes radially outward so as to hold the tubular member by contacting the inner peripheral surface of the tubular member.

4. The guide wire according to any one of claims 1 to 3, wherein the bulge portion is provided in a region from the distal end to the proximal end of the holding portion in a longitudinal direction.

5. The guide wire according to any one of claims 1 to 3, wherein the lubricious coating layer has at least one of a distal coating portion covering the distal end of the holding portion or a proximal coating portion covering the proximal end of the holding portion in a lumen of the tubular member.

6. The guide wire according to any one of claims 1 to 3, wherein the tubular member has a gap portion that allows communication between the outer peripheral surface of the tubular member and a lumen at at least one of a position adjacent to the distal end of the holding portion or a position adjacent to the proximal end of the holding portion.

7. The guide wire according to any one of claims 1 to 3, wherein the tubular member is a coil formed by winding a wire member so as to cover the outer periphery of the core member.

8. The guide wire according to claim 7, wherein the coil has a loosely wound portion at at least one of a position adjacent to the distal end of the holding portion and a position adjacent to the proximal end of the holding portion.

9. The guide wire according to any one of claims 1 to 3, wherein
the tubular member includes a distal-side tubular member and a proximal-side tubular member, and
the holding portion holds a proximal portion of the distal-side tubular member and a distal portion of the proximal-side tubular member.
